# NEW EUROPEAN PATENT SPECIFICATION

(11) **EP 3 090 780 B2**
(45) Date of publication and mention of the opposition decision: **25.05.2022**
(45) Mention of the grant of the patent: 13.03.2019
(21) Application number: 16165019.7
(22) Date of filing: 13.04.2016
(51) Int. Cl.: A61Q 19/00, A61K 8/97, A61K 8/81, A61K 8/04, A61K 8/06, A61K 8/9789

(54) **PROCESS FOR THE PREPARATION OF AN OIL-IN-WATER EMULSION**
PROZESS FÜR DIE HERSTELLUNG EINER ÖL-IN-WASSER EMULSION
PROCÈS POUR PRODUIRE UNE ÉMULSION HUILE DANS L'EAU

(30) Priority: 05.05.2015 IT RM20150195
(43) Date of publication of application: 09.11.2016
(73) Proprietor: DIVA BRANDS & PATENTS S.r.l., 06034 Foligno (PG) (IT)
(72) Inventor: Cocchioni, Pasquale, I-06034 Foligno (Perugia) (IT)
(74) Representative: Carangelo, Pierluigi

(56) References cited:
- EP-A1- 2 474 296
- WO-A1-2006/024095
- DE-A1-102008 059 445

## Description

### Field of the technique of the invention

This invention relates to a process for the preparation of an oil-in-water emulsion, usable in particular for the preparation of cosmetic products in the form of creams or lotions.

### State of the art

Among cosmetic products for daily use, compositions in the form of oil-in-water emulsions, such as lotions or creams, are widespread. These cosmetic products can have various functions, such as emollient, soothing, refreshing and cleansing functions, and so on.

The preparation of an oil-in-water emulsion requires a laborious and uneconomical process, especially when high volumes are involved. In fact, the emulsion is formed at a high temperature (50 to 80 °C), for which the energy expended to heat the oily phase and the aqueous phase is significant and economically Disadvantageous. WO2006024095 discloses a process for the preparation of an oil-in-water microemulsion or submicron emulsion composition for dermal delivery.

### Summary of the invention

One purpose of this invention is to provide a process that allows preparing an oil-in-water emulsion, in particular for the preparation of cosmetic products, which is efficient, give results at least comparable to those obtainable with conventional procedures and is more cost effective.

An object of this invention is therefore a process for preparing an oil-in-water emulsion as outlined in the appended claims.

Further characteristics and advantages of the process according to the invention will be evident from the following description of examples of preferred embodiments, provided in an indicative and non-limiting way.

### Detailed description of the invention

The process for the preparation of an oil-in-wa-ter emulsion according to claim 1 comprises the following steps:
i) preparation by heating of a solution or suspension of at least one emulsifier in a first aliquot of water;
ii) preparation by heating of an oily component comprising oils and optionally waxes;
iii) hot mixing of the oily component according to step ii) with the solution or suspension according to step i) obtaining a primary emulsion;
iv) mixing of said primary emulsion according to step iii) with a second aliquot of water at low temperature, obtaining said oil-in-water emulsion.

Said first aliquot of water in step i) is between 10% and 35%, preferably between 15% and 25%, of the water required for the final emulsion. Consequently, the second aliquot of water of step iv) is between 90% and 65%, preferably between 85% and 75%, of the water required for the final emulsion.

In certain embodiments, step i) is conducted at a temperature between 40 °C and 80 °C, preferably be-tween 50 °C and 70 °C. In certain embodiments, the mix-ture is stirred for a time of between 0.25 and 1.5 hours, preferably between 0.5 and 1 hour.

The at least one emulsifier is selected from sodium salts of acyl glutamates, salts of acyl taurates and salts of acyl sarcosinates.

In any case, it is a characteristic of this invention that the emulsifier system contains no derivative of the reaction of fatty acids with ethylene oxide or propylene oxide.

In certain embodiments, step ii) is conducted at a temperature between 40 °C and 80 °C, preferably be-tween 50 °C and 70 °C.

In certain embodiments, the oily component comprises at least one compound selected from: fatty alcohols with C12-C22 chain, behenylic alcohol, cetyl al-cohol, stearyl alcohol, vegetal triglycerides, derivatives of polymethylsiloxane, hydrocarbons with C13-C30 chain, even branched, and ethers of fatty acids.

Step iii) is preferably carried out by means of turbo-emulsifier at a speed comprised between 500 and 3000 rpm, preferably between 1000 and 2000 rpm. In any case, the necessary steps will be taken to obtain an emulsion. The primary emulsion thus obtained is stirred at a temperature preferably between 30 °C and 50 °C, more preferably between 35 °C and 45 °C.

According to step iv), the second aliquot of water, corresponding to a percentage of between 90% and 65% of the total water contained in the composition, is preferably at a temperature between 7 °C and 25 °C, more preferably between 12.5 °C and 20 °C.

In certain embodiments, step iv) is conducted by emulsifying the second aliquot of water and the primary emulsion under stirring, for example by means of mechanical stirring with a blade, at a speed between 50 and 300 rpm, preferably between 100 and 200 rpm. The agitation is continued for a time preferably comprised between 10 and 80 minutes, more preferably between 25 and 50 minutes.

The oil-in-water emulsions that can be prepared by means of the process of the invention may contain further active ingredients or additives, even heat-sensitive, depending on the use and the type of effect desired.

For example, one can use vitamins, urea, essential oils, bisabolol, plant extracts, polysaccharides, allantoin or peptides.

In the case of water-soluble, even heat-sensitive products, the further active ingredient or additive is added in the cold aqueous step at the end of the process.

In the case of fat-soluble heat-sensitive products, the further active ingredient or additive will be added in the finished emulsion, by dispersion even through the use of a suitable solubiliser.

In the case of fat-soluble, not heat-sensitive products the further active ingredient or additive is added in step ii.

The invention achieves the above purposes by providing a process for the efficient and economical preparation of an oil-in-water emulsion.

The invention will now be further described by the following formulation examples. The compositions listed in the examples were prepared according to the process of the invention described above.

### Formulation examples

### Example 1- Moisturising cream

Final quantity 100 kg

### Primary emulsion

Components
OILS:

- Butyrospermum Parkii Butter 2 kg
- Behenylic alcohol 2 kg
- Hydrogenated polydecene 5 kg
- Sodium stearoyl glutamate 0.3 kg
- Water 15 kg

Melt the oils at 65 °C. Heat the water separately at 65 °C. Emulsify the oil with water for 15 minutes at 1500 rpm by means of turbine, then allow to cool to 43 °C.

### Secondary phase:

components

- Water 75.7 kg at room temperature
- Primary emulsion 24.3 kg

Disperse the primary emulsion in the water by stirring at 100 rpm.

### Reference Example

Final quantity 100 kg

### Primary emulsion

Components
OILS:

- Ethylhexyl stearate 2.0 kg
- Cetearyl alcohol 1.5 kg
- Caprylic capric triglyceride 5 kg
- Cetearyl glucoside 0.2 kg
- Polyglyceryl dipoliidrossistearato 0.2kg
- Glycerol 1.1 kg
- Water 15 kg

Melt the oils at 65 °C. Heat the water separately at 65 °C. Emulsify the oil with water for 15 minutes at 1500 rpm by means of turbine, then allow to cool to 40 °C.

### Secondary phase:

components

- Water 74 kg at room temperature
- Primary emulsion 26 kg

Disperse the primary emulsion in the water by stirring at 100 rpm.

## Claims

1. Process for the preparation of an oil-in-water emulsion for cosmetic use, comprising the following steps:
i) preparation by heating of a solution or suspension consisting of at least one emulsifier in a first aliquot of water, wherein said first aliquot of water is between 10% and 35% of the water required for the final emulsion;
ii) preparation by heating of an oily component comprising oils and optionally waxes;
iii) hot mixing of the oily component according to step ii) with the solution or suspension according to step i) obtaining a primary emulsion;
iv) mixing of said primary emulsion according to step iii) with a second aliquot of water at low temperature, obtaining said oil-in-water emulsion, wherein said second aliquot of water is between 90% and 65% of the water required for the final emulsion,
wherein the at least one emulsifier is selected from sodium salts of acyl glutamates, salts of acyl taurates and salts of acyl sarcosinates.

2. Process according to claim 1, wherein said first aliquot of water in step i) is between 15% and 25% of the water required for the final emulsion; and said second aliquot of water of step iv) is between 85% and 75% of the water required for the final emulsion.

3. Process according to claims 1 or 2, wherein step i) is conducted at a temperature between 40 °C and 80 °C, or between 50 °C and 70 °C.

4. Process according to any of claims 1 to 3, wherein step ii) is conducted at a temperature between 40 °C and 80 °C, or between 50 °C and 70 °C.

5. Process according to any of claims 1 to 4, wherein step iii) is conducted by means of turbo-emulsifier at a speed between 500 and 3000 rpm, or between 1000 and 2000 rpm, and the primary emulsion thus obtained is stirred at a temperature between 30 °C and 50 °C, or between 35 °C and 45 °C.

6. Process according to any of claims 1 to 5, wherein the second aliquot of water is at a temperature between 7 °C and 25 °C, or between 12.5 °C and 20 °C.

7. Process according to any of claims 1 to 6, wherein step iv) is conducted by emulsifying the second aliquot of water and the primary emulsion under stirring at a speed between 50 and 300 rpm, or between 100 and 200 rpm.

8. Process according to any of claims 1 to 7, in which the oily component comprises at least one compound selected from: fatty alcohols with C12-C22 chain, behenylic alcohol, cetyl alcohol, stearyl alcohol, vegetal triglycerides, derivatives of polymethylsiloxane, hydrocarbons with C13-C30 chain and ethers of fatty acids.

## Patentansprüche

1. Verfahren zur Herstellung einer Öl-in-Wasser-Emulsion für kosmetische Zwecke, das die folgenden Schritte aufweist:
i) Herstellung durch Erhitzen einer Lösung oder Suspension, die aus zumindest einem Emulgator in einem ersten aliquoten Teil Wasser besteht, wobei der erste aliquote Teil Wasser zwischen 10 % und 35 % des für die endgültige Emulsion benötigten Wassers beträgt;
ii) Herstellung durch Erhitzen einer öligen Komponente, die Öle und optional Wachse aufweist;
iii) Heißmischen der öligen Komponente gemäß Schritt ii) mit der Lösung oder Suspension gemäß Schritt i), wodurch eine primäre Emulsion erhalten wird;
iv) Mischen der primären Emulsion gemäß Schritt iii) mit einem zweiten aliquoten Teil Wasser bei niedriger Temperatur, um die Öl-in-Wasser-Emulsion zu erhalten, wobei der zweite aliquote Teil Wasser zwischen 90 % und 65 % des für die endgültige Emulsion benötigten Wassers beträgt,
wobei der zumindest eine Emulgator ausgewählt ist aus Natriumsalzen von Acylglutamaten, Salzen von Acyltauraten und Salzen von Acylsarcosinaten.

2. Verfahren gemäß Anspruch 1, bei dem der erste aliquote Teil Wasser bei Schritt i) zwischen 15% und 25% des für die endgültige Emulsion benötigten Wassers beträgt; und der zweite aliquote Teil Wasser bei Schritt iv) zwischen 85% und 75% des für die endgültige Emulsion benötigten Wassers beträgt.

3. Verfahren gemäß Anspruch 1 oder 2, bei dem Schritt i) bei einer Temperatur zwischen 40 °C und 80 °C oder zwischen 50 °C und 70 °C durchgeführt wird.

4. Verfahren gemäß einem der Ansprüche 1 bis 3, bei dem Schritt ii) bei einer Temperatur zwischen 40 °C und 80 °C oder zwischen 50 °C und 70 °C durchgeführt wird.

5. Verfahren gemäß einem der Ansprüche 1 bis 4, bei dem Schritt iii) mittels Turboemulgator bei einer Drehzahl zwischen 500 und 3000 U/min oder zwischen 1000 und 2000 U/min durchgeführt wird und die so erhaltene primäre Emulsion bei einer Temperatur zwischen 30 °C und 50 °C oder zwischen 35 °C und 45 °C gerührt wird.

6. Verfahren gemäß einem der Ansprüche 1 bis 5, bei dem der zweite aliquote Teil Wasser eine Temperatur zwischen 7 °C und 25 °C oder zwischen 12,5 °C und 20 °C aufweist.

7. Verfahren gemäß einem der Ansprüche 1 bis 6, bei dem Schritt iv) durch Emulgieren des zweiten aliquoten Teils Wasser und der primären Emulsion unter Rühren bei einer Drehzahl zwischen 50 und 300 U/min oder zwischen 100 und 200 U/min durchgeführt wird.

8. Verfahren gemäß einem der Ansprüche 1 bis 7, bei dem die ölige Komponente zumindest eine Verbindung aufweist, die ausgewählt ist aus: Fettalkoholen mit C12-C22-Kette, Behylenylalkohol, Cetylalkohol, Stearylalkohol, pflanzlichen Triglyceriden, Derivaten von Polymethylsiloxan, Kohlenwasserstoffen mit C13-C30-Kette und Ethern von Fettsäuren.

## Revendications

1. Procédé pour la préparation d'une émulsion huile dans eau pour un usage cosmétique, comprenant les étapes suivantes :
i) préparation par chauffage d'une solution ou suspension consistant en au moins un émulsifiant dans une première aliquote d'eau, dans lequel ladite première aliquote d'eau se situe entre 10% et 35% de l'eau requise pour l'émulsion finale;
ii) préparation par chauffage d'un composant huileux comprenant des huiles et facultativement des cires ;
iii) mélange à chaud du composant huileux selon l'étape ii) avec la solution ou suspension selon l'étape i) pour obtenir une émulsion principale ;
iv) mélange de ladite émulsion principale selon l'étape iii) avec une seconde aliquote d'eau à basse température, pour obtenir ladite émulsion huile dans eau, dans lequel ladite seconde aliquote d'eau se situe entre 90% et 65% de l'eau requise pour l'émulsion finale,
dans lequel l'au moins un émulsifiant est sélectionné parmi des sels de sodium de glutamates d'acyle, des sels de taurates d'acyle et des sels de sarcosinates d'acyle.

2. Procédé selon la revendication 1, dans lequel ladite première aliquote d'eau dans l'étape 1) se situe entre 15 % et 25 % de l'eau requise pour l'émulsion finale ; et ladite seconde aliquote d'eau de l'étape iv) se situe entre 85 % et 75 % de l'eau requise pour l'émulsion finale.

3. Procédé selon les revendications 1 ou 2, dans lequel l'étape i) est réalisée à une température entre 40 °C et 80 °C, ou entre 50 °C et 70 °C.

4. Procédé selon l'une quelconque des revendications 1 à 3, dans lequel l'étape ii) est conduite à une température entre 40 °C et 80 °C, ou entre 50 °C et 70 °C.

5. Procédé selon l'une quelconque des revendications 1 à 4, dans lequel l'étape iii) est conduite au moyen d'un turbo-émulsionneur à une vitesse entre 500 et 3 000 tr/m, ou entre 1 000 et 2 000 tr/m, et l'émulsion principale ainsi obtenue est agitée à une température entre 30 °C et 50 °C, ou entre 35 °C et 45 °C.

6. Procédé selon l'une quelconque des revendications 1 à 5, dans lequel la seconde aliquote d'eau est à une température entre 7 °C et 25 °C, ou entre 12,5 °C et 20 °C.

7. Procédé selon l'une quelconque des revendications 1 à 6, dans lequel l'étape iv) est conduite en émulsionnant la seconde aliquote d'eau et l'émulsion principale agitée à une vitesse entre 50 et 300 tr/m, ou entre 100 et 200 tr/m.

8. Procédé selon l'une quelconque des revendications 1 à 7, dans lequel le composant huileux comprend au moins un composé sélectionné parmi : des alcools gras ayant une chaîne en C12-C22, l'alcool bénénylique, l'alcool cétylique, l'alcool stéarylique, des triglycérides végétaux, des dérivés de polyméthylsiloxane, des hydrocarbures ayant une chaîne en C13 à C30 et des éthers d'acides gras.
